# EUROPÄISCHE PATENTSCHRIFT

(11) **EP 4 215 215 B1**
(45) Veröffentlichungstag und Bekanntmachung des Hinweises auf die Patenterteilung: **04.03.2026**
(21) Anmeldenummer: 23151880.4
(22) Anmeldetag: 17.01.2023
(51) Int. Cl.: A61L 2/10

(54) **VORRICHTUNG MIT FUNKTIONSMODUL ZUM ENTKEIMEN VON MEHRWEG-BEHÄLTERN**
DEVICE WITH FUNCTIONAL MODULE FOR STERILISING REUSABLE CONTAINERS
DISPOSITIF DOTÉ D'UN MODULE FONCTIONNEL POUR DÉSINFECTER DES RÉCIPIENTS RÉUTILISABLES

(30) Priorität: 21.01.2022 DE 102022101427
(43) Veröffentlichungstag der Anmeldung: 26.07.2023
(73) Patentinhaber: Hilberer, Franz, 78234 Engen (DE)
(72) Erfinder: Hilberer, Franz, 78234 Engen (DE)
(74) Vertreter: Patentanwälte und Rechtsanwalt Weiß, Arat & Partner mbB

(56) Entgegenhaltungen:
- EP-A1- 3 628 338
- EP-A1- 3 957 333
- WO-A1-2016/073463
- DE-A1- 102019 001 287
- US-A1- 2018 113 066

## Beschreibung

### Technisches Gebiet

Die Erfindung betrifft eine Vorrichtung mit Funktionsmodul zum Entkeimen von Mehrweg-Behältern nach dem Oberbegriff des Anspruchs 1.

### Stand der Technik

Im Einzelhandel werden vereinzelt Mehrweg-Behälter akzeptiert, die von den Kunden selbst mit Wasser und Reinigungsmittel gereinigt wurden. Dies verstößt jedoch gegen geltende Hygienevorschriften.

Die EP 2 705 858 A1 beschreibt einen Desinfektionstunnel, welcher mit einem UV-Licht - Desinfizierer ausgestattet ist. Der Desinfektionstunnel weist im Inneren ein Förderband auf. Es dient der Desinfizierung von Paketen und zum verpackenden von Lebensmitteln etc..

Die US 5,958,336 A beschreibt einen Sterilisationstunnel, wobei in dem Sterilisationstunnel eine UV-Sterilisierung erfolgen soll. Der Sterilisationstunnel kommt als Zwischenschritt oder Abschlussschritt eines Produktionsprozesses zum Einsatz.

In der DE 10 2019 121 621 A1 -Vorrichtung zum Entkeimen von Mehrweg-Behältern- wird eine Vorrichtung offenbart auf deren Grundlage die vorliegende Erfindung aufbaut.

Zum Stand der Technik wird ausserdem auf die EP 3 628 338 A1 verwiesen, die eine Vorrichtung zum Entkeimen von Mehrweg-Behältern offenbart.

Die US 2018/0113066 A1 beschriebt eine Desinfektionsvorrichtung in Form einer Schublade.

Die WO 2016/073 463 A1 beschreibt einen Dekontaminationszelt für Personen.

Zuletzt wird auf die EP 3 957 333 A1 verwiesen, die Desinfektionskiste offenbart, deren vorgesehener Innenraum desinfiziert wird.

### Aufgabe der Erfindung

Die Aufgabe der vorliegenden Erfindung ist es, die Nachteile aus dem Stand der Technik zu überwinden. Insbesondere soll eine Vorrichtung bereitgestellt werden, die eine Reinigung, Wartung und den Austausch von Verschleißteilen erleichtert und komfortabler macht. Weiterhin soll die Bedienfreundlichkeit für das Thekenpersonal, wie auch für den Kunden erleichtert werden. Zudem soll die Entkeimung der Mehrwegbehälter schneller und zuverlässiger erfolgen. Ferner sollen Produktinformationen und Werbung für den Kunden bereitgestellt werden können. Schließlich soll die Leistungsfähigkeit der UV-C-Desinfizierer erhöht werden.

### Lösung der Aufgabe

Zur Lösung der Aufgabe führen die Merkmale nach dem Anspruch 1.

Vorteilhafte Ausgestaltungen sind in den Unteransprüchen beschrieben.

Zum Einsparen von Einweg-Behältern, wie Plastiktüten nutzen die Kunden im Einzelhandel immer öfter Mehrweg-Behälter in Lebensmittelmärkten oder Metzgertheken oder anderen vergleichbaren Läden, in denen frische Lebensmittel verkauft werden. Dazu bringt der Kunde seinen eigenen Mehrweg-Behälter zum Befüllen der Frisch-Ware mit, um sie an den Bedientheken füllen zu lassen.

Was auf vielen Wochenmärkten schon angewandt und bei den umweltfreundlichen Kunden auch gerne genutzt wird, ist für die Lebensmittelmärkte aus hygienischen Gründen bisher nicht zulässig.

Doch um auch dem Kunden des Lebensmittelmarktes diese Möglichkeit zu geben, ist hier eine erfindungsgemässe Vorrichtung gezeigt, bei der der Kunde seinen mitgebrachten Mehrweg-Behälter von einer Kundenseite über eine erste Klappe in ein beispielsweise unter der Theken-Auslage eingebauten absolut dicht verschweißten Gehäuse mit einem Funktionsmodul und einem Übergabetunnel an den Verkäufer übergeben kann, wobei eine Entkeimung der dort eingestellten Mehrweg-Behälter erfolgt.

Eine erfindungsgemässe Vorrichtung mit Funktionsmodul zum Entkeimen von Mehrweg-Behältern weist ein Gehäuse auf, wobei in dem Gehäuse ein Funktionsmodul angeordnet ist, welches einen Übergabetunnel mit Transporteinrichtung aufweist.

Das Gehäuse mit dem Funktionsmodul weist eine erste Öffnung und eine zweite Öffnung auf. In dem Funktionsmodul sind ein oder mehrere UVC-Desinfizierer vorhanden. Das Gehäuse und das Funktionsmodul bestehen aus einem metallenen Werkstoff wie Edelstahl oder Kunststoff. Der oder die UVC-Desinfizierer dient dabei einer schnellen und nahezu vollständigen und praktischen Entkeimung der auf der Transportvorrichtung anzuordnenden Mehrweg-Behälter.

Der UVC-Desinfizierer ist in der Regel eine UV-C - Lichtquelle, wobei es sich bei der UV-C - Lichtquelle um eine LED-Lampe handeln. Die LED-Lampe hat den Vorteil, dass sie energiesparend ist und sehr viel langlebiger als die üblichen UV-C - Birnen bzw. UV-C - Glühbirnen ist. Dies ist gerade vor dem Hintergrund bedeutend, dass in der Vorrichtung der UVC-Desinfizierer nach jedem Durchlauf eines Mehrweg-Lebensmittelbehältnisses zur Entnahme ausgeschaltet und anschließend nach Eingabe eines weiteren Mehrweg-Lebensmittelbehältnisses wieder eingeschaltet wird.

Weiterhin weist der UVC-Desinfizierer einen Querstromlüfter auf. Dieser Querstromlüfter kühlt die LED-UVC-Lampe und sorgt dabei dafür, dass diese leistungsfähig bleibt und nicht überhitzt. Dazu ist der Querstromlüfter automatisch temperaturgesteuert, was bedeutet, dass dieser bei einer vorgegebenen Temperatur mit der Luftkühlung beginnt. Die Temperatur, ab der der Querstromlüfter beginnt zu arbeiten, ist individuell programmierbar.

Weiterhin ist ein Temperatursensor angeordnet, welcher die Temperatur im Bereich des UVC-Desinfizierer erfasst und an den Querstromlüfter und/oder an einen internen Rechner übermittelt.

Der UVC-Desinfizierer gibt ein spezielles UVC Licht (also Ultraviolettes Licht) ab. Mehrweg-Behälter, welche von dem UVC-Desinfizierer mit UVC-Licht bestrahlt werden, werden desinfiziert, wobei es zur Abtötung von Bakterien/Keime oder dergleichen kommt.

Das Funktionsmodul ist in dem Gehäuse angeordnet. Dabei ist das Funktionsmodul vollständig aus dem Gehäuse ausfahrbar und wieder einfahrbar. Dazu ist das Funktionsmodul auf Schwerlastauszügen gelagert, welche es erlauben das Funktionsmodul aus einer der beiden Öffnungen des Gehäuses auszufahren.

Über beide Öffnungen ein- und ausfahrbar bedeutet, dass das Funktionsmodul sowohl an der ersten Öffnung des Gehäuses in Richtung der Kundenseite, als auch an der zweiten gegenüberliegenden Öffnung des Gehäuses in Richtung einer Bedienseite ein- und ausfahrbar ist. In bevorzugten Ausführungsformen ist das Funktionsmodul nur aus der ersten Öffnung in Richtung der Kundenseite ausfahrbar.

Dies bring die enormen Vorteile mit sich, dass das Funktionsmodul wie auch das Gehäuse auf einfache Weise gereinigt und gewartet werden können. Auch können Verschleißteile einfach und ohne einen aufwendigen Ausbau der Vorrichtung aus dem Gehäuse und dem Frischethekenbereich ersetzt werden.

Weiterhin ist eine abschließbare Verriegelung vorgesehen, die sicherstellt, dass das Funktionsmodul nur von berechtigtem Fachpersonal aus dem Gehäuse aus und wieder in dieses eingefahren werden kann.

Das Funktionsmodul stellt daher eine wesentliche Verbesserung dar. Insbesondere wird die regelmäßig vorzunehmende Reinigung durch das ausfahrbare Funktionsmodul enorm erleichtert, was dazu führt, dass ehemals schwer erreichbare Bereich nun leicht und schnelle gereinigt werden können.

Das Funktionsmodul weist dem Übergabetunnel auf, welcher einerseits über eine erste Klappe und andererseits über eine zweite Klappe zugänglich ist. In dem Übergabetunnel ist die Transporteinrichtung angeordnet. Die erste Klappe ist auf einer Kundenseite angeordnet, die zweite gegenüberliegende Klappe ist auf einer Bedienseite angeordnet ist.

Weiterhin weist das Funktionsmodul innerhalb des Übergabetunnels einen oder mehrere der UVC-Desinfizierer sowie einen ersten und zweiten Sensor auf.

Die erste Klappe des Funktionsmoduls und/oder die erste Öffnung des Gehäuses weisen einen ersten Sensor und die zweite Klappe des Funktionsmoduls und/oder die zweite Öffnung des Gehäuses weisen einen zweiten Sensor auf, wobei es sich bei den beiden Sensoren um eine Sicherungseinrichtung handelt und der oder die UVC-Desinfizierer nur bei geschlossenen Klappen bzw. bei durch das Funktionsmodul verschlossenen Öffnungen in Betrieb nehmbar sind.

Die zu desinfizierenden Mehrweg-Behälter werden auf der Kundenseite durch die geöffnete erste Klappe gereicht und auf der Transporteinrichtung platziert. Nachdem beide Klappen geschlossen wurden, transportiert die Transporteinrichtung die dort platzierten und zu desinfizierenden Mehrweg-Behälter von der ersten Klappe zu der zweiten Klappe. Auf diesem Weg wird der Mehrweg-Behälter von den UVC-Desinfizierer bestrahlt und dadurch desinfiziert.

Damit die zu desinfizierenden Mehrweg-Behälter von allen Seiten durch die in dem Funktionsmodul angeordneten UVC-Desinfizierer bestrahlt werden können, weist die Transporteinrichtung eine tablettförmige Quarzglasauflage auf. Die tablettförmige Quarzglasauflage erlaubt, dass die auf ihr angeordneten Mehrweg-Behälter von allen Seiten mit UV-C Strahlung bestrahlt werden können, da die Strahlung durch die tablettförmige Quarzglasauflage hindurchstrahlt. Die Quarzglasauflage kann natürlich auch aus einem anderen Material gefertigt sein, wesentlich ist jedoch, dass die UV-C Strahlen nicht an ihrer Transmission gehindert werden.

Die tablettförmige Quarzglasauflage ist einerseits oder beidseits, also orthogonal zu den gegenüberliegenden beiden Klappen, mit einem oder zwei Zahnriemenantrieb/en wirkverbunden. Ist nur auf einer Seite ein entsprechender Zahnriemenantrieb angeordnet, ist auf der gegenüberliegenden Seite eine Führungsschiene angeordnet, welche die diesseitige Aufnahme und Führung der Quarzglasauflage übernimmt. Es kann auch vorgesehen sein, dass sowohl Führungsschienen als auch Zahnriemenantrieb zusammen auf einer oder beiden Seiten angeordnet sind.

Der oder die Zahnriemenantrieb/e erlauben es, die tablettförmige Quarzglasauflage zwischen den beiden Klappen hin und her fahren zu lassen, wobei eine umfängliche Bestrahlung mit UV-C Strahlung durch die UVC-Desinfizierer erfolgen kann.

Die beiden Klappen des Funktionsmoduls weisen zumindest ein Sicherheitsscharnier auf, welches ein Öffnen der Klappe nur erlaubt, wenn die UVC-Desinfizierer nicht arbeiten und kein UV-C-Strahlung im Übergabetunnel vorhanden ist. Diese Sicherheitsscharniere sind mit den Sensoren in dem Funktionsmodul wirkverbunden.

Weiterhin weist das Funktionsmodul auf der Kundenseite, welche die Seite ist, auf der sich ein Kunde vor einer Frischetheke befindet, ein Display auf. Dieses Display sollen dem Kunden Informationen über die Verwendung der Vorrichtung und auch weitere Informationen, wie Produktinformationen und Werbung, übermittelt werden können.

Weiterhin sind an der Kundenseite Signalleuchten angeordnet die dem Kunden durch unterschiedliche Licht- und Farbsignale, wie beispielsweise grünes oder rotes Leuchten signalisieren, ob und wann die Vorrichtung geöffnet und beladen werden kann.

Weiterhin ist auf einer Bedienseite des Funktionsmoduls vorgesehen, dass dort ein Hauptschalter für die In- und Außerbetriebnahme, ein Notausschalter, ein Startknopf sowie Betriebsanzeigeleuchten angeordnet sind. Die Bedienseite ist die Seite einer Frischetheke, auf der Bedienpersonal den Kunden bedient und Zugriff auf die in der Frischetheke platzierten Waren hat.

Ferner ist vorgesehen, dass die Vorrichtung ein Netzteil mit vorzugsweise 230 V über einen Kaltgeräteschalter und -stecker umfasst. Das Netzteil ist vorzugsweise auf der Bedienseite angeordnet. Natürlich können hier auch andere übliche Netzanschlüsse vorgesehen sein.

Zudem verfügt das Funktionsmodul an der Bedienseite über mehrere Anschlüsse bzw. Schnittstellen, wie USB- Anschlüsse oder dergleichen. Diese Anschlüsse bzw. Schnittstellen erlauben das Anschließen vor externen Geräten, wie Rechnern oder dergleichen. Über diese Schnittstellen, kann auf das Display und einen intern verbauten Rechner zugegriffen werden und Informationen abgerufen und/oder übertragen werden.

Natürlich kann auch vorgesehen sein, dass das Display ein Touchscreen ist, welcher für das Personal freigeschaltet werden kann. Ein solcher Touchscreen erlaubt den gesicherten Zugriff auf ein internes Steuerungsmenü in welchem Einstellungen und Wartungen vorgenommen werden können.

Ferner ist das Innere des Übergabetunnels mit einer UV-reflektierenden Oberfläche ausgestattet, so dass die UV-C Strahlung reflektiert wird.

Der oder die UVC-Desinfizierer sind mit dem ersten und dem zweiten Sensor sowie den Sicherheitsscharnieren der beiden Kappen wirkverbunden.

Die tablettförmige Quarzglasauflage ist über den Zahnriemenantrieb mit dem Funktionsmodul verbunden. In dem Übergabetunnel des Funktionsmoduls sind die UVC-Desinfizierer angeordnet. Diese sind vorzugsweise an einer Decke und an einem Boden des Übergabetunnels angeordnet ist, wobei ein UVC-Desinfizierer von der Decke zu dem Boden ausgerichtet ist und ein weiterer UVC-Desinfizierer vom dem Boden zur Decke ausgerichtet ist. Die tablettförmige Quarzglasauflage, auf der die Mehrwegbehälter platziert werden, ermöglicht es, dass die UV-C- Stahlen die Mehrwegbehälter von allen Seiten erreichen. Zuvor musste auf umständliche Weise erreicht werden, dass ein Förderband unterbrochen wurde, so dass auch die Standflächen der Mehrwegbehälter für kurze Zeit Bestrahlt werden konnten. Dies hat regelmäßig zu Problemen geführt, welche nun nicht mehr auftreten.

Die erfindungsgemäße Vorrichtung ist derart gestaltet, dass das Gehäuse als Teil einer Ladentheke, oder Kühltheke ausgebildet sein kann. Natürlich ist es auch vorgesehen, dass die Vorrichtung als separates Beistellgerät neben einer Kühltheke oder an einem anderen Ort platziert werden kann.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in:
- Figur 1: eine seitliche Ansicht der erfindungsgemässen Vorrichtung V auf eine Bedienseite 3;
- Figur 2: eine seitliche Ansicht der erfindungsgemässen Vorrichtung V auf eine Kundenseite 21 mit ausgefahrenem Funktionsmodul 2;
- Figur 3: eine von der Bedienseite 3 hin zur Kundenseite 21 geschnittene Ansicht der Vorrichtung V mit Blick auf die Transporteinrichtung 4.

### Ausführungsbeispiel

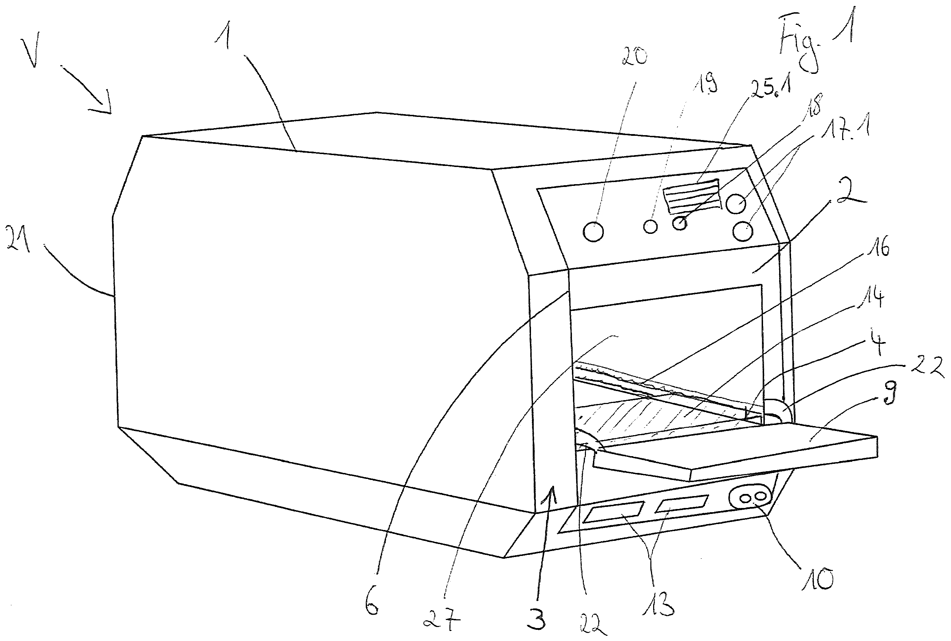

In Figur 1 ist eine seitliche Ansicht der erfindungsgemässen Vorrichtung V mit Blick auf eine Bedienseite 3 dargestellt. In dieser Ansicht ist ein Funktionsmodul 2 in ein Gehäuse 1 eingefahren.

Das Gehäuse 1 bildet eine umfassende Aufnahme für des Funktionsmodul 2 aus. Dabei weist das Gehäuse 1 eine erste Öffnung 5 und eine gegenüberliegende zweit Öffnung 6 auf. Beide Öffnungen sind in Figur 1 durch das in das Gehäuse 1 eingefahrene Funktionsmodul 2 verschlossen.

Das Funktionsmodul 2 weist einen Übergabetunnel 27 auf, welcher einends, auf einer Kundenseite 21 von einer erste Klappe 8 und andernends, auf einer Bedienseite 3, von einer zweite Klappe 9 verschlossen wird.

Die zweite Klappe 9 des Funktionsmoduls 2 ist in vorliegender Figur 1 geöffnet dargestellt, so dass der Blick in den Übergabetunnel 27 des Funktionsmoduls 2 freigegeben ist. Der Übergabetunnel 27 erstreckt sich von der ersten Klappe 8, die an der Kundenseite 21 der Vorrichtung V angeordnet ist hin zu einer zweiten Klappe 9, die an der Bedienseite 3 der Vorrichtung V angeordnet ist.

Im Übergabetunnel 27 ist eine Transporteinrichtung 4 angeordnet. Diese Transsporteinrichtung 4 besteht aus einer tablettförmigen Quarzglasauflage 14, welche mit einem Zahnriemenantrieb 16 wirkverbunden ist.

Weiterhin sind an der Bedienseite 3 des Funktionsmoduls 2, oberhalb der zweiten Klappe 9, Anzeigeleuchten 17, ein Startknopf 18, ein Notausknopf 19 und ein Hauptschalter 20 angeordnet. Zudem sind ebenfalls oberhalb der Klappe 9 Lüftungsschlitze 25.1 angeordnet. Unterhalt der zweiten Klappe 9 sind Schnittstellen 13, beispielsweise USB-Anschlüsse sowie ein Netzteilanschluss 10 angeordnet.

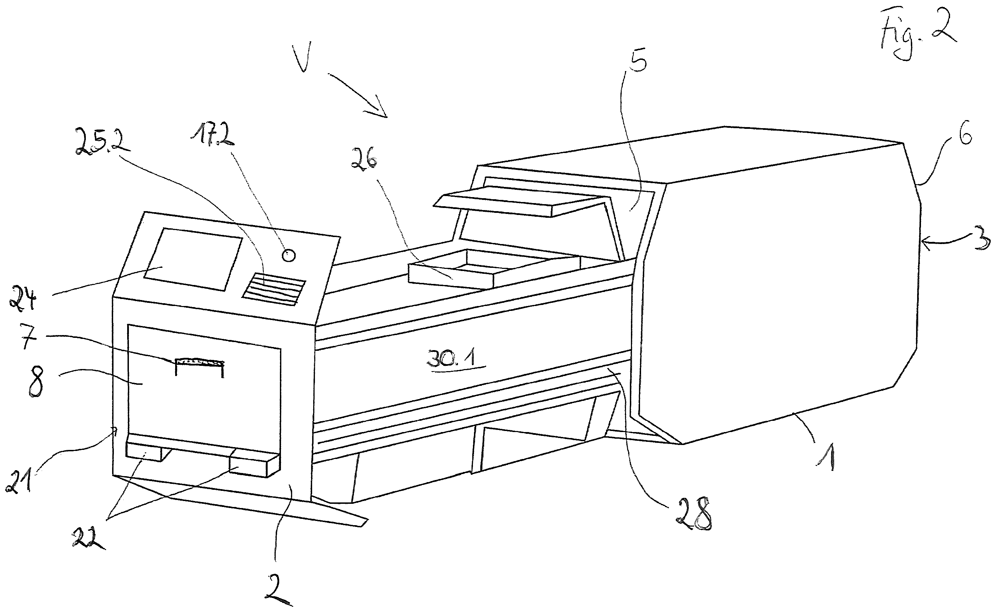

In Figur 2 ist eine seitliche Ansicht der erfindungsgemässen Vorrichtung V auf eine Kundenseite 21 dargestellt, wobei das Funktionsmodul 2 aus der Öffnung 5 des Gehäuses 1 ausgefahren ist. In dieser Ansicht wird deutlich, dass das Funktionsmodul 2, welches den überwiegenden Teil der wesentlichen Bestandteile der Vorrichtung V enthält, auf einfache Weise gereinigt und gewartet werden kann.

Weiterhin kann vorgesehen sein, dass eine erste Seitenwand 30.1 und/oder eine gegenüberliegende zweite Seitenwand 30.2 des Übergabetunnels 27 in Teilen oder auch vollständig aufklappbar ist. Dies hat den Vorteil, dass das Innere des Übergabetunnels 27 in der Position des ausgefahrenen Funktionsmoduls 2 noch leichter erreicht werden kann und Reparatur- und insbesondre Reinigungsarbeiten leicht vorgenommen werden können.

Die Funktionseinheit 2 ist beidseitig, nach dem Vorbild von Schubladenauszügen, auf Schwerlastauszügen 28 gelagert und mit dem Gehäuse 1 verbunden. Die Schwerlastauszüge 28 erlauben es die gesamte Funktionseinheit 2 aus der ersten Öffnung 5 des Gehäuses 1 herauszufahren und dort auch wieder hineinzufahren.

Natürlich kann auch vorgesehen sein, dass das Funktionsmodul 2 in die gegenüberliegende Richtung, also in Richtung der Bedienseite 3 aus einer zweiten Öffnung 6 des Gehäuses 1 herausfahrbar und wieder einfahrbar ist. Vorgesehen ist auch, dass das Funktionsmodul 2 sowohl in Richtung der Bedienseite 3 als auch in Richtung der Kundenseite 21 aus dem Gehäuse 1 heraus- und einfahrbar ist.

An der Kundenseite 21 des in Figur 2 aus dem Gehäuse 1 ausgefahrenen Funktionsmoduls 2 ist oberhalb der ersten Klappe 8 ein Display 24 angeordnet. Weiterhin sind neben dem Display 24 ein weitere Anzeigeleuchte 17.2 sowie weitere Lüftungsschlitze 25.2 angeordnet. Natürlich können die Größen dieser Elemente variieren, ebenso wie die Positionierung auf dem Funktionsmodul 2 und die Anordnung zueinander.

Die erste Klappe 8 ist in Figur 2 in einer geschlossenen Position dargestellt. Sie verfügt über einen Griff 7. Dabei sind zwei am unteren Ende der ersten Klappe 8 angeordnete Sicherheitsscharniere 22 ersichtlich. Diese Sicherheitsscharniere erlauben das Öffnen der ersten Klappe 8 nur, wenn durch ein Sensorsignal bestätigt wird, dass in dem Übergabetunnel keine UV-C Strahlung mehrvorhanden ist.

Weiterhin ist eine Luftkühlung in Form eines Querstromlüfters 26 an dem Funktionsmodul 2 angeordnet. Der Querstromlüfter 26 verfügt zudem über einen Temperatursensor, welcher hier nicht ersichtlich ist. Der Querstromlüfter 26 dient zur rückseitigen Kühlung der in dem Übergabetunnel 27 angeordneten UVC-Desinfizierer.

Über die Lüftungsschlitze 25.1, wird an der Bedienseite 3 durch die Querstromlüfter 26 Raumluft angesogen, welche dann über die Lüftungsschlitze 25.2 an der Kundenseite 21 wieder ausgeblasen wird, nachdem die LEDs der UVC-Desinfizierer gekühlt wurden. Die Strömungsrichtung ist hierbei von Bedeutung, so dass die zur Kühlung verwendete Luft nicht in den Bereich der Bedienseite 3 gelangen kann.

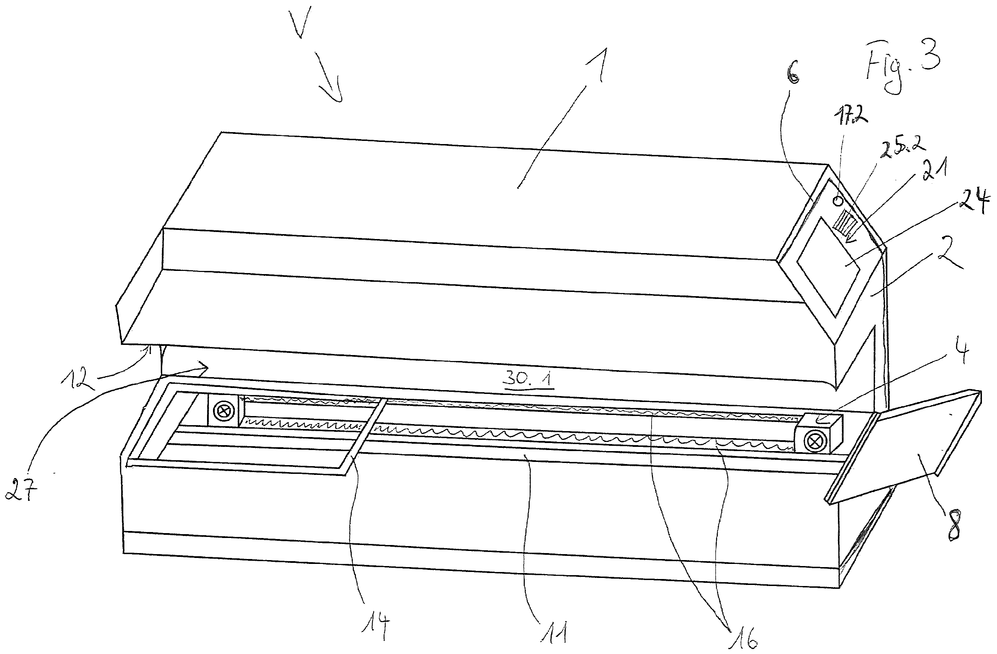

In Figur 3 ist eine, von der Bedienseite 3 hin zur Kundenseite 21, also entlang des Übergabetunnels 27, geschnittene Ansicht der Vorrichtung V mit Blick auf die Transporteinrichtung 4 dargestellt.

In Figur 3 ist die Funktionseinheit 2 in das Gehäuse 1 der Vorrichtung V eingefahren. Innerhalb des Übergabetunnels 27 ist die Transporteinrichtung 4 ersichtlich, welche aus der tablettförmigen Quarzglasauflage 14 besteht, die mit dem Zahnriemenantrieb 16 wirkverbunden ist. Hier ist auch ersichtlich, dass die Quarzglasauflage 14 durchsichtig ist, so dass die UV-C Strahlen der UVC-Desinfizierer, die innerhalb des Übergabetunnels 27 angeordnet sind, einen auf der Quarzglasauflage 14 zur Desinfizierung platzierte Mehrwegbehälter von jeder Seite aus erreichen können.

Weiterhin sind an der Kundenseite 21 des Funktionsmoduls 2 das Lüftungsschlitze 25.2, ein Teil des Displays 24 sowie Anzeigeleuchten 17.2 ersichtlich, welche bereits beschrieben wurden.

Bezugnehmend auf die Figuren 1 bis 3 erklärt sich die Funktionsweise der erfindungsgemässen Vorrichtung V in folgenden Schritten:

### 1. Schritt:

Ein Kunde öffnet die erste Klappe 8 und legt seinen mitgebrachten Mehrweg-Behälter und die dazugehörigen Behälterdeckel getrennt von den Mehrweg-Behältern auf die Quarzglasauflage 14 der Transporteinrichtung 4. Natürlich können die Mehrweg-Behälter und die dazugehörigen Deckel auch getrennt voneinander desinfiziert werden. Die Anordnung auf der Quarzglasauflage 14 richtet sich vornehmlich nach der Größe des zu desinfizierenden Behältnisses und kann nach Wunsch verändert werden.

### 2. Schritt:

Der Kunde schließt die erste Klappe 8. Das Bedienpersonal setzt durch den Startknopf 18 die Transporteinrichtung 4 in Bewegung, wobei auch eine automatische Inbetriebnahme bei Schließen beider Klappen 8, 9 möglich sein kann. Dabei werden gleichzeitig die UVC-Desinfizierer im verschlossenen Übergabetunnel 27 eingeschaltet, wobei der UVC-Desinfizierer nur eingeschaltet werden, wenn die beiden Sensoren detektieren, dass die beiden Öffnungen 5, 6 des Gehäuses 1 durch die Funktionseinheit 2 verschlossen und die beiden Klappen 8, 9, die den Zugang zum Übergabetunnel 27 freigeben, verschlossen sind.

### 3. Schritt:

Die Mehrweg-Behälter werden auf der Quarzglasauflage 14, welche durch den Zahnriemenantrieb 16 bewegt wird, im Übergabetunnel 27 von der ersten Klappe 8 in Richtung der zweiten Klappe 9 bewegt, also von der Kundenseite 21 in Richtung der Bedienseite 3. Dieser Vorgang soll ca. 10 Sekunden dauern, kann jedoch aus kürzer oder länger sein. Dabei werden die Mehrweg-Behälter von den eingeschalteten UVC-Desinfizierern mit UV-C-Strahlung bestrahlt, wobei die Quarzglasauflage 14 in Verbindung mit der UV-reflektierenden Oberfläche des Übergabetunnels 27 gewährleistet, dass die Mehrweg-Behälter und die entsprechenden Deckel von allen Seiten bestrahlt werden können und so zuverlässig desinfiziert werden.

### 4. Schritt:

Das Bedienpersonal stoppt mit dem Druckschalter die Transporteinrichtung 4 und deaktiviert die UVC-Desinfizierer. Dies kann auch automatisch erfolgen, sobald die Quarzglasauflage 14 die zweite Klappe 9 erreicht hat.

### 5. Schritt:

Das Bedienpersonal öffnet die zweite Klappe 9 und entnimmt die Mehrweg-Behälter und die Deckel von der Quarzglasauflage 14 der Transporteinrichtung 4 und befüllt die Mehrweg-Behälter mit den Bestellungen des Kunden.

### 6. Schritt:

Das Bedienpersonal fährt am Ende eines Arbeitstages das Funktionsmodul 2, welches durch ein Schloss mit dem Gehäuse 1 verbunden ist, über die Schwerlastauszüge 28 aus dem Gehäuse 1 heraus, so dass eine Reinigung vorgenommen werden kann. Dazu können die Seitenwandungen 30.1 und 30.2 des Übergabetunnels 27 teilweise oder auch vollständig aufgeklappt werden, so dass ein Zugang in den Übergabetunnel leicht möglich ist. Zudem ist die Quarzglasauflage 14 zu Reinigungszwecken von der Transporteinrichtung 4 bzw. dem Zahnriemenantrieb 16 entnehmbar. Das Schloss kann über einen weiteren Sensor verfügen, welcher erfasst, ob UV-C-Strahlung vorhanden ist. Wenn dies der Fall ist, lässt sich das Schloss erst dann öffnen, wenn der Sensor keine UV-C-Strahlung mehr erfasst. Dies ist eine zusätzliche Sicherungsmaßnahme.

### Schritt 7:

Nach erfolgter Reinigung, wird das Funktionsmodul 2 wieder in das Gehäuse 1 eingefahren und dort verriegelt.

### Schritt 8:

Über den Anschluss eines Rechners über die Schnittstellen 13 kann das Bedienpersonal auf den internen Rechner zugreifen und festlegen, welche Abbildungen und Texte auf dem Display 24 dem Kunden angezeigt werden sollen. Weiterhin können so auch andere Einstellung vorgenommen werden.

### Bezugszeichenliste

| | |
|---|---|
| 1 | Gehäuse |
| 2 | Funktionsmodul |
| 3 | Bedienseite |
| 4 | Transporteinrichtung |
| 5 | erste Öffnung |
| 6 | zweite Öffnung |
| 7 | Griff |
| 8 | erste Klappe |
| 9 | zweite Klappe |
| 10 | Netzteil |
| 11 | Boden |
| 12 | Decke |
| 13 | Schnittstelle |
| 14 | Quarzglasauflage |
| | |
| 16 | Zahnriemenantrieb |
| 17 | Anzeigeleuchten |
| 18 | Startknopf |
| 19 | Notausschalter |
| 20 | Hauptschalter |
| 21 | Kundenseite |
| 22 | Sicherheitsscharnier |
| 23 | |
| 24 | Display |
| 25 | Lüftungsschlitze |
| 26 | Querstromlüfter |
| 27 | Übergabetunnel |
| 28 | Schwerlastzug |
| | |
| 30.1 | erste Seitenwand |
| 30.2 | zweite Seitenwand |
| V | Vorrichtung |

## Patentansprüche

1. Vorrichtung (V) zum Entkeimen von Mehrweg-Behältern mit einem Gehäuse (1), ein oder mehreren UVC-Desinfizierer/n und einer Transporteinrichtung (4), wobei das Gehäuse (1) eine erste Öffnung (5) und/oder eine zweite Öffnung (6) aufweist, wobei der UVC-Desinfizierer eine UV-C - Lichtquelle ist, wobei die UV-C - Lichtquelle eine LED-Lampe ist, und wobei eine erste Klappe (8) und eine zweite Klappe (9) angeordnet ist, wobei die erste Klappe (8) und/oder die erste Öffnung (5) einen ersten Sensor umfasst/en und die zweite Klappe (9) und/oder die zweite Öffnung (6) einen zweiten Sensor umfasst/en, wobei es sich bei den beiden Sensoren um eine Sicherungseinrichtung handelt und der UVC-Desinfizierer nur bei geschlossenen Klappen (8, 9) in Betrieb nehmbar ist, wobei die Mehrweg-Behälter auf der Transporteinrichtung (4) zu der ersten Klappe (8) oder zu der zweiten Klappe (9) bewegbar sind,
**dadurch gekennzeichnet,**
**dass** die Transporteinrichtung (4), die beiden Klappen (8,9), die UVC-Desinfizierer sowie der erste und zweite Sensor in einem Funktionsmodul (2) angeordnet sind, wobei das Funktionsmodul (2) aus dem Gehäuse (1) aus- und einfahrbar ist.

2. Vorrichtung nach Anspruch 1, **dadurch gekennzeichnet, dass** die Transporteinrichtung (4) eine tablettförmige Quarzglasauflage (14) aufweist, die mit einem Zahnriemenantrieb (16) wirkverbunden ist.

3. Vorrichtung nach Anspruch 1, **dadurch gekennzeichnet, dass** der UVC-Desinfizierer einen Querstromlüfter (26) aufweist.

4. Vorrichtung nach Anspruch 1, **dadurch gekennzeichnet, dass** die Klappen (8, 9) jeweils ein Sicherheitsscharnier (22) aufweisen.

5. Vorrichtung nach Anspruch 1, **dadurch gekennzeichnet, dass** auf einer Kundenseite (21) ein Display (24) und Anzeigeleuchten (17.1) angeordnet sind, und wobei auf einer Bedienseite (3) ein Hauptschalter (20), ein Notausschalter (19), ein Startknopf (18) sowie weitere Anzeigeleuchten (17.2) angeordnet sind.

6. Vorrichtung nach einem der vorigen Ansprüche, **dadurch gekennzeichnet, dass** das Innere des Funktionsmoduls (2) und/oder das Innere eines Übergabetunnels (27) eine UV-C-reflektierende Oberfläche aufweist.

7. Vorrichtung nach einem der vorigen Ansprüche, **dadurch gekennzeichnet, dass** die UVC-Desinfizierer mit dem ersten und dem zweiten Sensor und den Sicherheitsscharnieren (22) wirkverbunden sind.

8. Vorrichtung nach einem der vorigen Ansprüche, **dadurch gekennzeichnet, dass** eine Quarzglasauflage (14) über einen Zahnriemenantrieb (16) mit einem Übergabetunnel (27) des Funktionsmoduls (2) verbunden ist und ein UVC-Desinfizierer an einer Decke (12) und ein weiterer UVC-Desinfizierer an einem Boden (11) des Übergabetunnels (27) des Funktionsmoduls (2) angeordnet ist, wobei der UVC-Desinfizierer (7) von der Decke (12) zu dem Boden (11) ausgerichtet ist und ein weiterer UVC-Desinfizierer vom dem Boden (11) zur Decke (12) ausgerichtet ist.

9. Vorrichtung nach einem der vorigen Ansprüche, **dadurch gekennzeichnet, dass** das Funktionsmodul (2) eine auf der Bedienseite (3) und der Kundenseite (21) Lüftungsschlitze (25) aufweist.

10. Vorrichtung nach einem der vorigen Ansprüche, **dadurch gekennzeichnet, dass** das Funktionsmodul (2) Schwerlastauszüge (28) aufweist, über die das Funktionsmodul (2) aus dem Gehäuse (1) aus- und einfahrbar ist, wobei dies sowohl an der ersten Öffnung (5) des Gehäuses (1) in Richtung der Kundenseite (21), als auch an der zweiten Öffnung (6) des Gehäuses (1) in Richtung der Bedienseite (3) möglich ist.

## Claims

1. Device (V) for sterilising reusable containers, comprising a housing (1), one or more UVC disinfectors and a transport device (4), wherein the housing (1) has a first opening (5) and/or a second opening (6), wherein the UVC disinfector is a UV-C light source, wherein the UV-C light source is an LED lamp, and wherein a first flap (8) and a second flap (9) are arranged, wherein the first flap (8) and/or the first opening (5) comprise a first sensor and the second flap (9) and/or the second opening (6) comprise a second sensor, wherein the two sensors are a safety device and the UVC disinfector can only be put into operation when the flaps (8, 9) are closed, wherein the reusable containers can be moved on the transport device (4) to the first flap (8) or to the second flap (9),
**characterised in**
**that** the transport device (4), the two flaps (8, 9), the UVC disinfectors and the first and second sensors are arranged in a functional module (2), wherein the functional module (2) can be extended and retracted from the housing (1).

2. Device according to claim 1, **characterised in that** the transport device (4) has a tablet-shaped quartz glass support (14) which is operatively connected to a toothed belt drive (16).

3. Device according to claim 1, **characterised in that** the UVC disinfector has a cross-flow fan (26).

4. Device according to claim 1, **characterised in that** the flaps (8, 9) each have a safety hinge (22).

5. Device according to claim 1, **characterised in that** a display (24) and indicator lights (17.1) are arranged on a customer side (21), and wherein a main switch (20), an emergency stop switch (19), a start button (18) and further indicator lights (17.2) are arranged on an operator side (3).

6. Device according to one of the preceding claims, **characterised in that** the interior of the function module (2) and/or the interior of a transfer tunnel (27) has a UV-C-reflecting surface.

7. Device according to one of the preceding claims, **characterised in that** the UVC disinfectors are operatively connected to the first and second sensors and the safety hinges (22).

8. Device according to one of the preceding claims, **characterised in that** a quartz glass support (14) is connected to a transfer tunnel (27) of the functional module (2) via a toothed belt drive (16) and a UVC disinfector is arranged on a ceiling (12) and a further UVC disinfector is arranged on a floor (11) of the transfer tunnel (27) of the functional module (2), wherein the UVC disinfector (7) is aligned from the ceiling (12) to the floor (11) and a further UVC disinfector is aligned from the floor (11) to the ceiling (12).

9. Device according to one of the preceding claims, **characterised in that** the functional module (2) has ventilation slots (25) on the operating side (3) and the customer side (21).

10. Device according to one of the preceding claims, **characterised in that** the function module (2) has heavy-duty pull-out rails (28) via which the function module (2) can be pulled out of the housing (1), whereby this is possible both at the first opening (5) of the housing (1) in the direction of the customer side (21) and at the second opening (6) of the housing (1) in the direction of the operating side (3).

## Revendications

1. Dispositif (V) pour désinfecter des récipients réutilisables, comprenant un boîtier (1), un ou plusieurs désinfecteurs UVC et un moyen de transport (4), le boîtier (1) présentant une première ouverture (5) et/ou une deuxième ouverture (6), le désinfecteur UVC étant une source de lumière UV-C, la source de lumière UV-C étant une lampe à DEL, et il est prévu un premier volet (8) et un deuxième volet (9), le premier volet (8) et/ou la première ouverture (5) comprenant un premier capteur, et le deuxième volet (9) et/ou la deuxième ouverture (6) comprenant un deuxième capteur, les deux capteurs constituant un moyen de sécurité, et le désinfecteur UVC ne pouvant être mis en service que lorsque les volets (8, 9) sont fermés, les récipients réutilisables pouvant être déplacés sur le moyen de transport (4) vers le premier volet (8) ou vers le deuxième volet (9),
**caractérisé en ce que**
le moyen de transport (4), les deux volets (8, 9), les désinfecteurs UVC ainsi que les premier et deuxième capteurs sont disposés dans un module fonctionnel (2), le module fonctionnel (2) pouvant être sorti du boîtier (1) et y être rentré.

2. Dispositif selon la revendication 1,
**caractérisé en ce que** le moyen de transport (4) comporte un support en verre de quartz (14) en forme de plateau qui est relié de manière opérationnelle à un entraînement à courroie crantée (16).

3. Dispositif selon la revendication 1,
**caractérisé en ce que** le désinfecteur UVC comporte un ventilateur à flux transversal (26).

4. Dispositif selon la revendication 1,
**caractérisé en ce que** les volets (8, 9) comportent chacun une charnière de sécurité (22).

5. Dispositif selon la revendication 1,
**caractérisé en ce qu'**un écran d'affichage (24) et des voyants lumineux (17.1) sont disposés sur un côté client (21), et un interrupteur principal (20), un interrupteur d'arrêt d'urgence (19), un bouton de démarrage (18) ainsi que d'autres voyants lumineux (17.2) sont disposés sur un côté commande (3).

6. Dispositif selon l'une des revendications précédentes,
**caractérisé en ce que** l'intérieur du module fonctionnel (2) et/ou l'intérieur d'un tunnel de transfert (27) présente une surface réfléchissant les UV-C.

7. Dispositif selon l'une des revendications précédentes,
**caractérisé en ce que** les désinfecteurs UVC sont reliés de manière opérationnelle au premier et au deuxième capteur et aux charnières de sécurité (22).

8. Dispositif selon l'une des revendications précédentes,
**caractérisé en ce que** le support en verre de quartz (14) est relié à un tunnel de transfert (27) du module fonctionnel (2) par l'intermédiaire d'un entraînement à courroie crantée (16), et un désinfecteur UVC est disposé au plafond (12) et un autre désinfecteur UVC est disposé au fond (11) du tunnel de transfert (27) du module fonctionnel (2), le désinfecteur UVC (7) étant orienté du plafond (12) vers le fond (11), et un autre désinfecteur UVC étant orienté du fond (11) vers le plafond (12).

9. Dispositif selon l'une des revendications précédentes,
**caractérisé en ce que** le module fonctionnel (2) présente des fentes d'aération (25) sur le côté commande (3) et sur le côté client (21).

10. Dispositif selon l'une des revendications précédentes,
**caractérisé en ce que** le module fonctionnel (2) comprend des glissières pour charges lourdes (28) qui permettent au module fonctionnel (2) de sortir du boîtier (1) et d'y rentrer, et ce aussi bien au niveau de la première ouverture (5) du boîtier (1) en direction du côté client (21) qu'au niveau de la deuxième ouverture (6) du boîtier (1) en direction du côté commande (3).
